# EUROPEAN PATENT APPLICATION

(11) **EP 2 682 150 A1**
(43) Date of publication of application: **08.01.2014**
(21) Application number: 13175027.5
(22) Date of filing: 04.07.2013
(51) Int. Cl.: A61M 16/20, F17D 3/01, G06Q 50/06, G06Q 50/22

(54) **Methods and apparatus for controlling fluid flow in medical facilities**

(30) Priority: 04.07.2012 US 201261667987 P
(71) Applicant: Hunt, Barry W, Kitchener, Ontario N2P 2L2 (CA); Jarrett, Todd Eric, Burlington, Ontario L7P 0H1 (CA); Over, Christopher B, Cambridge, Ontario N3C 0B9 (CA); Thorne, Damian Ross, Kitchener, Ontario N2P 1P1 (CA); Elgadi, Osama, Kitchener, Ontario N2C 2P6 (CA); Martinez Vazquez, Cesar Laurentino, Cambridge, Ontario N3C 3M8 (CA)
(72) Inventor: Hunt, Barry W, Kitchener, Ontario N2P 2L2 (CA); Jarrett, Todd Eric, Burlington, Ontario L7P 0H1 (CA); Over, Christopher B, Cambridge, Ontario N3C 0B9 (CA); Thorne, Damian Ross, Kitchener, Ontario N2P 1P1 (CA); Elgadi, Osama, Kitchener, Ontario N2C 2P6 (CA); Martinez Vazquez, Cesar Laurentino, Cambridge, Ontario N3C 3M8 (CA)
(74) Representative: Cordina, Kevin John

(57) **Abstract**

An apparatus for controlling fluid flow in a medical facility includes a plurality of remotely controlled zone valves. Each zone valve is coupled to a respective fluid conduit for opening and closing the fluid conduit to control fluid flow therethrough. The apparatus also includes an access terminal installed in a user-accessible location that is located remotely from the zone valves. For example, the zone valve may be located in a ceiling, and the access terminal may be located on a wall. The access terminal has an input interface for receiving instructions to open and close the zone valves, an output device for communicating with the zone valves to remotely open and close each zone valve based on the received instructions, and an alarm for indicating when at least one of the zone valves is closed.

## Description

### Technical Field

Embodiments disclosed herein relate to controlling fluid flow in medical facilities, and, in particular, to apparatus and methods for controlling fluid flow to particular zones of a medical facility using zone valves.

### Introduction

Hospitals and other medical facilities normally have pipes or fluid conduits for supplying fluids such as medical gases and vacuum suction to patients. For various reasons, at times it may be desirable to shut-off flow of these fluids to particular regions or zones within the medical facility. For example, gas and vacuum supply lines may be shut-off when performing routine maintenance, or during a fire.

Traditional zone valves use manually operated valves that are installed in user accessible locations (e.g., on a wall within a hallway in the medical facility). Piping for each fluid is routed to each corresponding zone valve. For example, main gas and vacuum supply lines are usually located in the ceiling of a medical facility. To connect these main supply lines to the zone valves, pipes are installed within the walls and extend down from the main supply lines to the zone valves and then back up to the main supply lines. Installing this piping can be time-consuming and expensive. This piping can also occupy valuable space within the wall that could be used for other purposes.

In some countries, regulations exist that govern the use of zone valves. Specifically, some regulations require zone valves to have an indicator that identifies the status of the valve as being open or closed.

### Summary of Some Embodiments

According to one aspect there is provided a method for controlling fluid flow in a medical facility. The method includes receiving instructions to control fluid flow through at least one fluid conduit to at least one zone in the medical facility. The instructions are received at a user-accessible location that is located remotely from the at least one fluid conduit. The method further includes remotely operating a zone valve coupled to the at least one fluid conduit to open and close the at least one fluid conduit based on the received instructions, and activating at least one alarm when the at least one zone valve is closed.

According to another aspect there is provided an apparatus for controlling fluid flow in a medical facility. The apparatus includes a plurality of remotely controlled zone valves, each zone valve being coupled to a respective fluid conduit for opening and closing the fluid conduit to control fluid flow therethrough. The apparatus also includes an access terminal installed in a user-accessible location that is located remotely from the zone valves. The access terminal has an input interface for receiving instructions to open and close the zone valves, an output device for communicating with the zone valves to remotely open and close each zone valve based on the received instructions, and an alarm for indicating when at least one of the zone valves is closed.

According to yet another aspect there is provided a method of installing an apparatus for controlling fluid flow in a medical facility. The method includes coupling at least one zone valve to a respective fluid conduit, the zone valve being adapted to selectively open and close the respective fluid conduit for controlling fluid flow to at least one zone in the medical facility. The method also includes installing an access terminal within the medical facility at a user-accessible location that is located remotely from the zone valve. The access terminal has an input interface for receiving instructions to open and close the zone valves, an output device for communicating with the zone valves to remotely open and close each zone valve based on the received instructions, and an alarm for indicating when at least one of the zone valves is closed.

### Brief Description of the Drawings

The drawings included herewith are for illustrating various examples of articles, methods, and apparatuses of the present specification and are not intended to limit the scope of what is taught in any way. In the drawings:

FIG. 1 is a bottom perspective view of an apparatus for controlling fluid flow in a medical facility according to one embodiment;

FIG. 2 is an enlarged bottom perspective view of a portion of the apparatus of FIG. 1 showing a housing for enclosing a plurality of zone valves;

FIG. 3 is a partially sectioned top perspective view of the housing of FIG 2;

FIG. 4 is a schematic diagram of the apparatus of FIG. 1;

FIGS. 5A and 5B are schematic wiring diagrams of the apparatus of FIG. 1;

FIG. 6 is a flow chart depicting a method of controlling fluid flow in a medical facility according to another embodiment; and

FIG. 7 is a flow chart depicting a method of installing an apparatus for controlling fluid flow in a medical facility according to yet another embodiment.

### Description of Various Embodiments

It will be appreciated that numerous specific details are set forth in order to provide a thorough understanding of the exemplary embodiments described herein. However, it will be understood by those of ordinary skill in the art that the embodiments described herein may be practiced without these specific details. In other instances, well-known methods, procedures and components have not been described in detail so as not to obscure the embodiments described herein. Furthermore, this description is not to be considered as limiting the scope of the embodiments described herein in any way, but rather as merely describing implementation of the various embodiments described herein.

In some cases, the embodiments described herein may be implemented in hardware, software, firmware, or a combination thereof. In some cases, embodiments may be implemented in one or more computer programs executing on one or more programmable computing devices comprising at least one processor, a data storage device (including in some cases volatile and nonvolatile memory and/or data storage elements), at least one input device, and at least one output device.

In some embodiments, each program may be implemented in a high level procedural or object oriented programming and/or scripting language to communicate with a computer system. However, the programs can be implemented in assembly or machine language, if desired. In any case, the language may be a compiled or interpreted language.

In some embodiments, the systems and methods as described herein may also be implemented as a non-transitory computer-readable storage medium configured with a computer program, wherein the storage medium so configured causes a computer to operate in a specific and predefined manner to perform at least some of the functions as described herein.

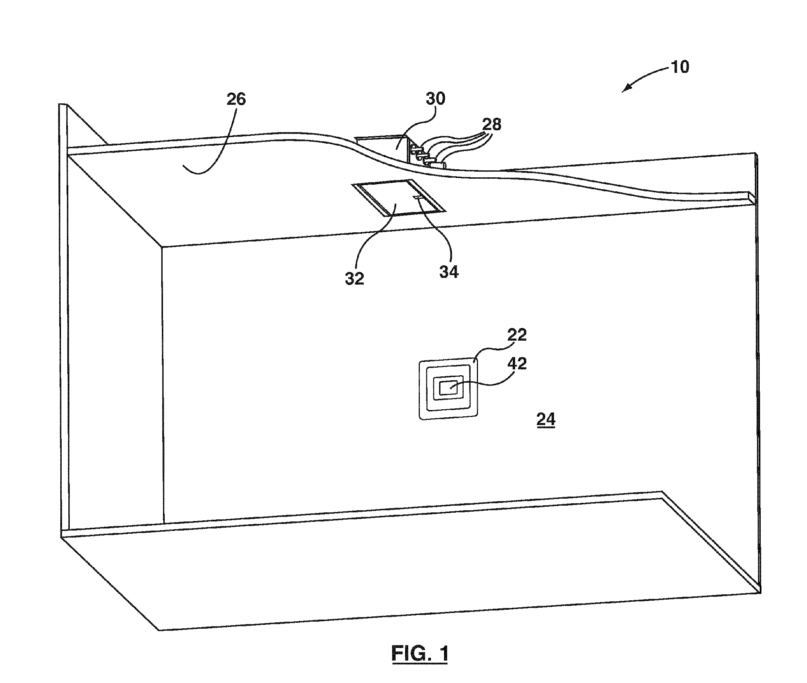

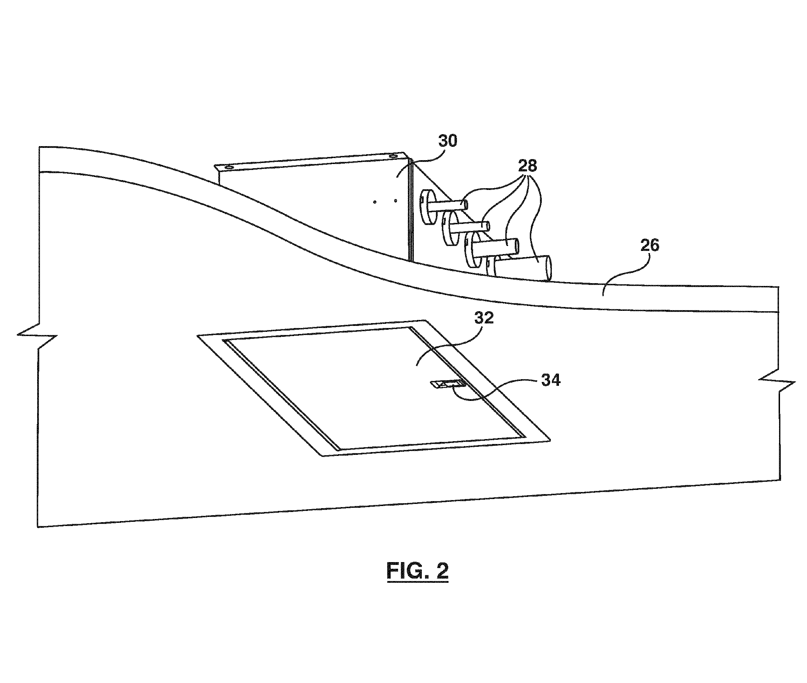

Referring now to FIGS. 1-3, illustrated therein is an apparatus 10 for controlling fluid flow in a medical facility according to one embodiment. The apparatus 10 generally includes a plurality of remotely controlled zone valves 20 (e.g. within a housing 30) and an access terminal 22 for remotely operating the valves 20.

Each zone valve 20 is coupled to a corresponding fluid conduit 28. Generally speaking, each the zone valve 20 can be used to open and close its corresponding fluid conduit 28 to control fluid flow therethrough.

The fluid conduits 28 may be pipes or other conduits that supply fluids such as medical gases, for example oxygen, nitrogen and air. The fluid conduits 28 could also provide a source of suction, such as a medical vacuum. The fluid conduits 28 are normally located above a ceiling 26 in a medical facility, but in some cases could be located in other places (e.g., within the walls or floors of the medical facility).

The access terminal 22 is generally adapted to be installed in a user-accessible location that is located remotely from the zone valves 20. For example, the access terminal 22 may be located on a wall 24 within the medical facility, with the zone valves 20 coupled to the fluid conduits 28 above the ceiling 26. In other examples, the access terminal 22 and the valves 20 may have other locations. For example, the access terminal 22 could be located at a centralized location (e.g., a nursing station).

The access terminal 22 generally receives instructions to remotely open and close the zone valves 20. Installing the access terminal 22 remotely from the zone valves 20 and using remote operation can reduce the time and expense associated with installing fluid conduits throughout the medical facility. For example, remote installation and operation can allow the zone valves 20 to be installed in-line with existing fluid conduits that are located within the ceiling 26 while still allowing operation of the zone valves 20 from a user-accessible location such as at a wall within the medical facility.

Referring still to FIG. 1, in some embodiments the zone valves 20 may be located within a housing 30, which may enclose the valves 20. The housing 30 may be installed in the ceiling 26. The housing 30 may include an access cover 32 such as a door that allows a user to access the zone valves 20 and other components within the housing 30. The access cover 32 may be held closed by a latch 34.

The housing 30 may help protect the valves 20. For example, the housing 30 may have fire retardant properties or may have a water-resistant seal to protect the valves 20 in the event of a fire or a water leak.

The housing 30 can also help organize the zone valves 20. For example, the housing 30 may provide a single access point for a group of valves. This may be useful when conducting maintenance or when manually overriding the valves 20 (e.g. using a manual override such as a lever), which may in some embodiments allow all the valves 20 in a particular housing 30 to be quickly identified and closed.

In other embodiments, the valves 20 may be installed without the housing 30. For example, each valve 20 may be coupled to each respective fluid conduit 28 as a standalone unit. This standalone installation may allow the zone valves to be installed directly in-line whereas use of the housing 30 may require re-routing of some fluid conduits. This may be beneficial in a retrofit scenario to avoid having to reroute existing piping to connect with the housing 30.

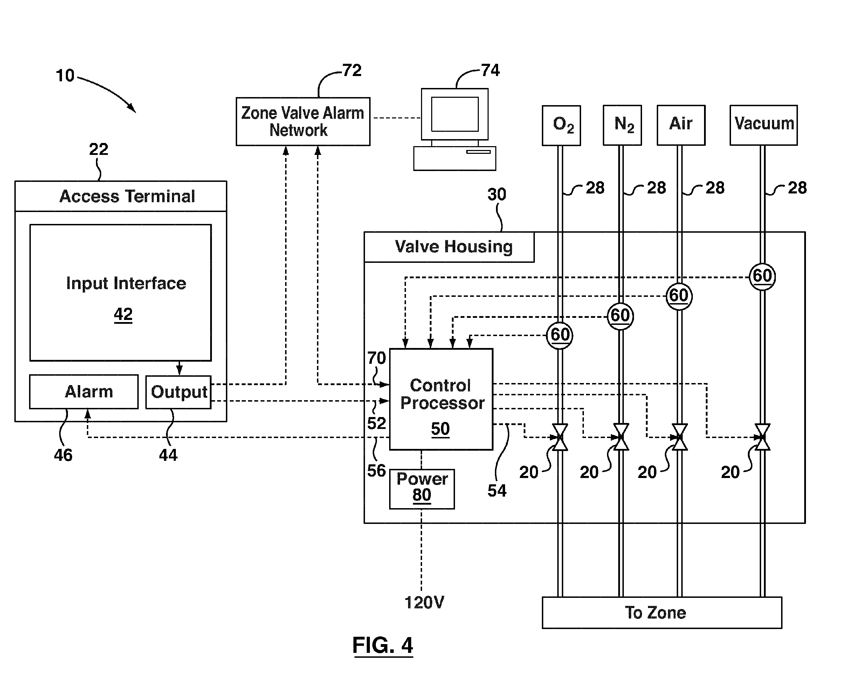

Referring now to FIG. 4, the access terminal 22 generally receives instructions from a user to remotely open and close the zone valves 20. Accordingly, the access terminal 22 generally includes an input interface 42 for receiving instructions to open and close the zone valves 20. The input interface 42 generally allows a user to enter instructions to open or close one or more valves. For example, the input interface 42 may include a touch screen display, a computer terminal, and the like. In some embodiments, the input interface 42 may include various other input devices, such as a keyboard, a mouse, one or more physical buttons, and so on. In some embodiments, the input interface 42 may include wireless communications via a portable electronic device, such as a smartphone or dedicated personal data assistant. For example, maintenance personnel may desire to control the access terminal 22 using a dedicated application on a smartphone.

The access terminal 22 also includes an output device 44 for remotely communicating with the zone valves 20, which could generally be done via a wired connection (e.g., Ethernet, coaxial cable, etc.) or a wireless connection (e.g., Bluetooth, WIFI, etc.).

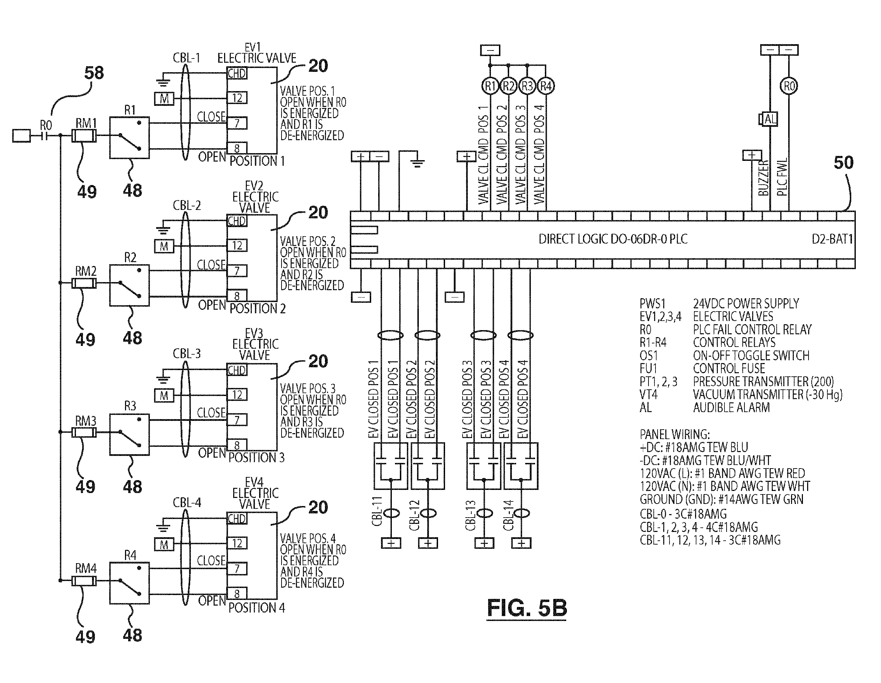

The output device 44 may directly or indirectly operate the zone valves 20 based on the received instructions. For example, with reference to FIG. 5B, the output device 44 may indirectly communicate with the zone valves 20 through a valve actuator device to control the valves 20. In some embodiments, the valve actuator device may include electromechanical components such as a relay 48 and an electric motor coupled to a ball valve (e.g. a ¼ turn ball valve). Furthermore, each relay 48 may be protected from over-current by a fuse 49, circuit breaker, or other circuit isolation device.

In other embodiments, the valve actuator could include other mechanical or electromechanical components such as solenoids.

In yet other embodiments, each zone valve 20 may have its own built-in valve actuator device for opening and closing the valve 20. This may allow the output device 44 to directly communicate with zone valves 20.

As shown, the access terminal 22 also includes an alarm 46 for indicating when at least one of the zone valves 20 is closed. In some embodiments, the alarm 46 may provide an audio alarm (e.g. via a speaker), a visual alarm (e.g. via a display or blinking light), some other type of alarm (e.g., tactile vibration), or a combination thereof.

In some embodiments, the apparatus 10 may also include at least one control processor 50. The control processor 50 may facilitate communication between the output device 44 and the valves 20. The control processor 50 may include an access terminal input 52, one or more valve control outputs 54, and an alarm control output 56.

As shown, the access terminal input 52 is operatively coupled to the output device 44 of the access terminal 22. The access terminal input 52 generally receives information from the output device 44 corresponding to the instructions received (by the input interface 42) to open and close the zone valves 20. The access terminal input 52 may communicate with the output device 44 via a wired or wireless connection.

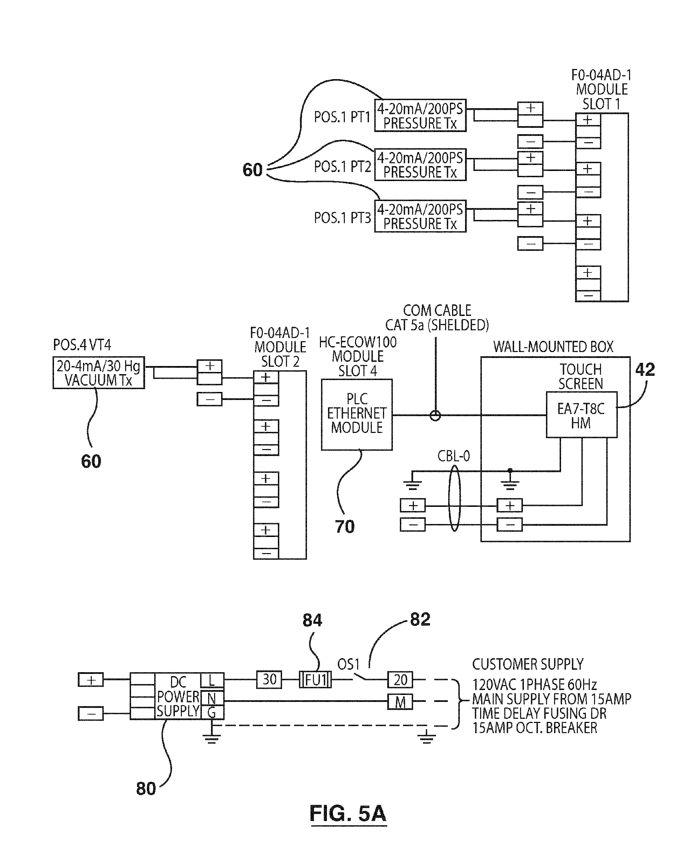

The valve control outputs 54 are operatively coupled to the zone valves 20 (e.g. using wired or wireless connections). Each valve control output 54 is in communication with each respective zone valve 20 to remotely open and close the respective zone valve 20 based on the instructions received from the access terminal 22. In some embodiments, as shown in FIG. 5B, a main relay 58 may be connected to the valve control outputs 54 to provide power to each of the individual zone valve relays 48.

The alarm control output 56 is operatively coupled to the alarm 46 (e.g. using a wired or wireless connection). The alarm control output 56 generally activates the alarm 46 when at least one of the zone valves 20 is closed.

As shown in FIG. 4, the at least one control processor 50 may be located remotely from the access terminal 22. For example, the control processor 50 may be located within the housing 30 near the zone valves 20. Alternatively, the control processor 50 may be integrated with the access terminal 22. In some embodiments, the control processor 50 could be located separate from both the access terminal 22 and the zone valves 20.

In some embodiments, the apparatus 10 may include one or more sensors 60 in communication with the control processor 50. Each sensor 60 may be coupled to a respective fluid conduit 28. The sensors 60 can be utilized to provide monitoring of fluid properties within the fluid conduits 28, in some cases, in real-time or substantially real-time. For example, the sensors 60 may include pressure sensors, flow sensors, temperature sensors, and the like.

In some embodiments, the sensors 60 may include pressure sensors for detecting fluid pressure within a respective fluid conduit 28. If the fluid pressure exceeds a particular threshold value, the control processor 50 may be configured to activate a second alarm. In some embodiments, the second alarm may be emitted from the same source as the first alarm 46 (e.g. using the same speaker, display or light). Alternatively, the second alarm may be a separate alarm.

In some embodiments, the apparatus 10 may include a network interface 70 for communicating with a zone valve alarm network 72. As shown in FIG. 4, in some embodiments the network interface 70 may be incorporated as part of the control processor 50. In other embodiments, the network interface 70 may be a separate component, or may be integrated as part of the access terminal 22.

In some embodiments, the zone valve alarm network 72 may allow communication between a plurality of apparatuses 10, and possibly with at least one server, within a medical facility. This may allow remote monitoring and control of the apparatuses 10 from one or more access points, such as the access terminals 22 or a centralized location (e.g., a nursing station, a maintenance room, a security desk, etc.).

The zone valve alarm network 72 may allow a computing device 74 to communicate with one or more of the apparatuses 10. This may allow the computing device 74 to remotely monitor or control operation of the zone valves 20. For example, the computing device 74 may be configured to monitor data associated with one or more of instructions received from the access terminal 22, a status of the zone valves 20 as being opened or closed, a status of the alarm 46, or some combination thereof.

In some embodiments, the computing device 74 may be configured to control one or more of the zone valves 20 through the zone valve alarm network 72. For example, the computing device 74 may provide instructions to the control processor 50 to remotely open and close the zone valves 20. As another example, the computing device 74 may remotely operate the with zone valves 20 by directly communicating with the zone valves 20 without using the control processor 50.

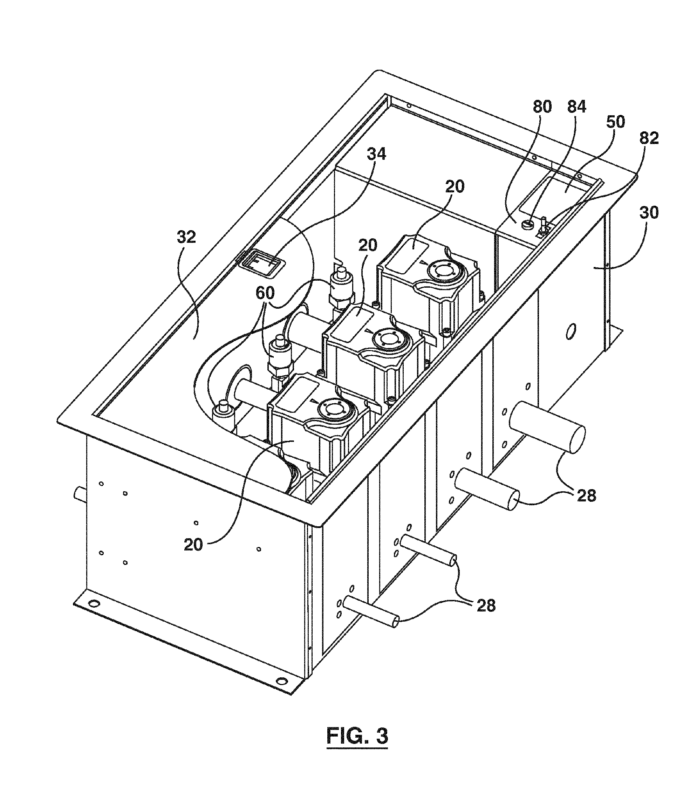

The apparatus 10 may also include a power supply 80 for powering one or more components of the apparatus 10 such as the valves 20, the access terminal 22, and the control processor 50. As shown in FIG. 3, in some embodiments the power supply 80 may be enclosed within the housing 30 and could be located adjacent to the control processor 50. In some embodiments, as shown in FIGS. 3 and 5A, the power supply 80 may also include a power switch 82 and a fuse 84.

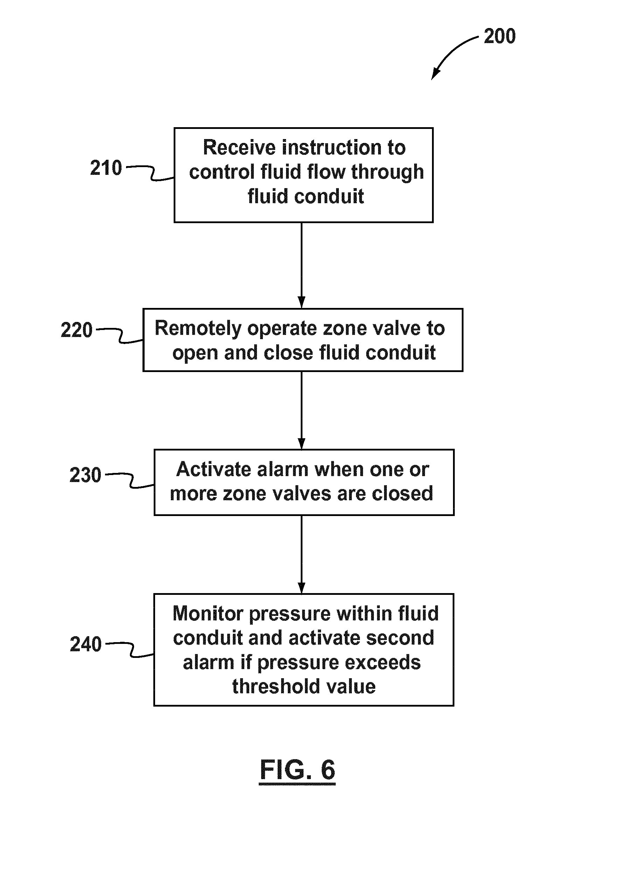

Referring now to FIG. 6, illustrated therein is a method 200 for controlling fluid flow in a medical facility according to another embodiment. The method 200 generally includes steps 210, 220, 230, and 240 although it is contemplated that other steps could also be performed.

Step 210 includes receiving instructions to control fluid flow through at least one fluid conduit to at least one zone in a medical facility. The instructions are received at a user-accessible location that is located remotely from the fluid conduit. For example, the instructions may be received at an access terminal (e.g., access terminal 22 located on a wall within the medical facility with the fluid conduit 28 being located in a nearby ceiling).

Step 220 includes remotely operating a zone valve coupled to the fluid conduit in order to open and close the fluid conduit based on the received instructions. For example, the instructions received at the access terminal 22 may be used to remotely operate one or more of the zone valves 20 positioned in the ceiling and coupled to the fluid conduits 28.

Step 230 includes activating at least one alarm when at least one of the zone valves is closed. For example, step 230 may include activating the alarm 46 when one of the zone valves 20 is closed.

In some embodiments, the instructions received at step 210 may be used to control fluid flow through a plurality of fluid conduits by remotely operating a plurality of zone valves. For example, the received instructions may control each of the zone valves 20 to open and close the respective fluid conduits 28.

Furthermore, in some embodiments, the instructions received at step 210 may be used to control fluid flow to a plurality of zones in the medical facility.

In some embodiments, the method 200 may also include a step 240 of monitoring fluid pressure within the at least one fluid conduit, and activating a second alarm when the fluid pressure exceeds a threshold value.

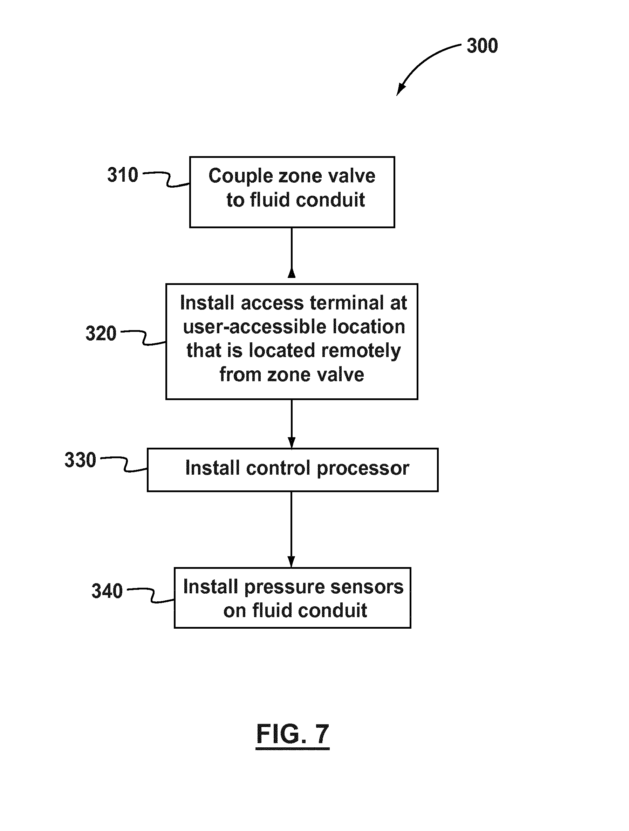

Referring now to FIG. 7, illustrated therein is a method 300 of installing an apparatus for controlling fluid flow in a medical facility such as the apparatus 10. The method generally includes steps 310, 320, 330 and 340, although it is contemplated that various other related steps could be performed.

Step 310 includes coupling at least one zone valve to a respective fluid conduit. The zone valve is adapted to selectively open and close fluid conduit for controlling fluid flow to at least one zone in the medical facility. For example, step 310 may include coupling one or more of the zone valves 20 to the respective fluid conduits 28. In some embodiments, this may be done in a retrofit context, where the zone valves 20 are installed in an existing medical facility and are coupled to existing medical supply lines. In other embodiments, this may be done in a new installation context, in which the zone valves 20 are installed generally around the same time as the medical supply lines.

In some embodiments, the zone valve may be coupled to the fluid conduit at a location behind a wall surface such as in a ceiling.

In some embodiments, step 310 may also include installing a housing for enclosing the zone valves, such as the housing 30 described above.

Step 320 includes installing an access terminal within the medical facility at a user-accessible location that is located remotely from the zone valve. For example, step 320 may include installing the access terminal on a wall within the medical facility, such as the access terminal 22 described above.

The method 300 may also include step 330 of installing a control processor such as the control processor 50. In some embodiments, the control processor may be installed integrally with the access terminal. In other embodiments, the control processor may be installed in a location remote from the access terminal.

The method 300 may also include step 340 of installing a plurality of sensors in communication with the control processor. For example, each sensor may be coupled to a respective fluid conduit so as to monitor fluid pressure therein. Furthermore, the control processor may be configured to activate a second alarm when the fluid pressure within the respective fluid conduit exceeds a threshold value.

While the steps of the above methods have been described sequentially hereinabove, it should be noted that sequential performance of the steps may not need to occur for successful implementation of the method. As will be evident to one skilled in the art, rearranging sequence of performance of the steps, omitting the performance of some steps, or performing the steps in parallel may be possible in various embodiments.

While the above description provides examples of one or more apparatus, methods, or systems, it will be appreciated that other apparatus, methods, or systems may be within the scope of the present description as interpreted by one of skill in the art.

## Claims

1. A method for controlling fluid flow in a medical facility, the method comprising:
a) receiving instructions to control fluid flow through at least one fluid conduit to at least one zone in the medical facility, the instructions being received at a user-accessible location that is located remotely from the at least one fluid conduit;
b) remotely operating a zone valve coupled to the at least one fluid conduit to open and close the at least one fluid conduit based on the received instructions; and
c) activating at least one alarm when the zone valve is closed.

2. The method of claim 1, wherein the instructions are received at an access terminal.

3. The method of claim 2, wherein the access terminal is located on a wall within the medical facility.

4. The method of any preceding claim, wherein the zone valve is located behind a wall surface.

5. The method of claim 4, wherein the zone valve is located within a ceiling.

6. The method of any preceding claim, wherein the received instructions are used to control fluid flow through a plurality of fluid conduits by remotely operating a plurality of zone valves, each of the zone valves corresponding to one of the fluid conduits.

7. An apparatus for controlling fluid flow in a medical facility, the apparatus comprising:
a) a plurality of remotely controlled zone valves, each zone valve being coupled to a respective fluid conduit for opening and closing the fluid conduit to control fluid flow therethrough; and
b) an access terminal installed in a user-accessible location that is located remotely from the zone valves, the access terminal comprising:
i) an input interface for receiving instructions to open and close the zone valves;
ii) an output device for communicating with the zone valves to remotely open and close each zone valve based on the received instructions; and
iii) an alarm for indicating when at least one of the zone valves is closed.

8. The apparatus of claim 7, further comprising a control processor that includes:
a) an access terminal input operatively coupled to the output device of the access terminal for receiving information corresponding to the received instructions to open and close the zone valves;
b) a plurality of valve control outputs operatively coupled to the zone valves, each valve control output being in communication with each respective zone valve to remotely open and close the respective zone valve based on the received instructions; and
c) an alarm control output operatively coupled to the alarm for activating the alarm when at least one of the zone valves is closed.

9. The apparatus of claim 8, further comprising a plurality of sensors in communication with the control processor, each sensor being coupled to a respective fluid conduit for monitoring fluid pressure therein, the control processor being configured to activate a second alarm when the fluid pressure within the respective fluid conduit exceeds a threshold value.

10. The apparatus of any one or more of claims 7 to 9, further comprising a network interface for communicating with a zone valve alarm network within the medical facility.

11. The apparatus of claim 10, wherein the network interface is configured to communicate with a computing device that is in communication with the zone valve alarm network to monitor data associated with at least one of:
the received instructions;
a status of the zone valves being opened or closed; and
a status of the alarm.

12. A method of installing an apparatus for controlling fluid flow in a medical facility, the method comprising:
a) coupling a zone valve to a respective fluid conduit, the zone valve being adapted to selectively open and close the respective fluid conduit for controlling fluid flow to at least one zone in the medical facility;
b) installing an access terminal within the medical facility at a user-accessible location that is located remotely from the zone valve, the access terminal comprising:
i) an input interface for receiving instructions to open and close the zone valves;
ii) an output device for communicating with the zone valves to remotely open and close each zone valve based on the received instructions; and
iii) an alarm for indicating when at least one of the zone valves is closed.

13. The method of claim 12, wherein the zone valve is coupled to the fluid conduit at a location behind a wall surface.

14. The method of any one or more claims 12 and 13, wherein the access terminal is installed on a wall within the medical facility.

15. The method of any one or more of claims 12 to 14, wherein the zone valve is coupled in-line with an existing fluid conduit.
